# EUROPEAN PATENT SPECIFICATION

(11) **EP 1 229 036 B1**
(45) Date of publication and mention of the grant of the patent: **12.01.2005**
(21) Application number: 01101521.1
(22) Date of filing: 24.01.2001
(51) Int. Cl.: C07D 405/12, C07D 311/58, A61K 31/453, A61P 5/32

(54) **2H-1-benzopyran derivatives, processes for their preparation and pharmaceutical compositions thereof**
2H-1-Benzopyran-Derivative, Prozesse zu ihrer Herstellung und Pharmazeutische Zusammensetzungen
Derivés de 2H-1-benzopyran, procédés pour leur preparation et compositions pharmaceutique les contenant

(43) Date of publication of application: 07.08.2002
(73) Proprietor: CHIESI FARMACEUTICI S.p.A., I-43100 Parma (IT)
(72) Inventor: Delcanale, Maurizio, 43100 Parma (IT); Amari, Gabriele, 43100 Parma (IT); Armani, Elisabetta, 43100 Parma (IT); Civelli, Maurizio, 43100 Parma (IT); Galbiati, Elisabetta, 43100 Parma (IT)
(74) Representative: Minoja, Fabrizio, Dr.

(56) References cited:
- WO-A-00/39120
- US-A- 5 280 040
- US-A- 5 985 306
- US-A- 6 153 768
- GRESE, T. A. ET AL.: "Structure-activity relationship of selective estrogen receptor modulators ..." J. MED. CHEM., vol. 40, 1997, pages 146-167, XP002053054

## Description

The present invention relates to 2H-1-benzopyran derivatives, processes for their preparation and their pharmaceutical compositions.

More precisely, the invention relates to 2H-1-benzopyran derivatives of general formula: 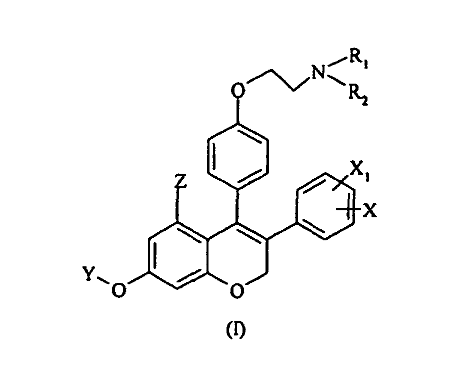 wherein:
- R₁ and R₂,: taken together with the nitrogen atom, form a piperidino or a pyrrolidino ring,
- Y: is hydrogen or a C₁-C₄ alkanoyl or a pivaloyl group,
- Z: is OR₄, wherein R₄ is H or C₁-C₄ alkyl,
- X and X₁: are hydrogen or one is hydrogen and the other is halogen or an OR₃ group wherein R₃ is H, C₁-C₄ alkyl, C₁-C₄ alkanoyl, pivaloyl or benzoyl.

The compounds of the invention can exist in the form of salts.

The compounds bearing basic groups can exist in the form of organic or inorganic acid addition salts.

The compounds bearing acid groups can exist in the form of addition salts with alkali or alkaline-earth metal or organic amines.

X and X₁ are hydrogen or one is hydrogen and the other is halogen or an OR₃ group as defined above, in the para position.

Particularly preferred are also the following compounds:
3-phenyl-4-(4-(2-(N-piperidinyl)ethoxy)phenyl)-5-hydroxy-7-pivaloyloxychrom-3-ene hydrochloride;
3-(4-methoxyphenyl)-4-(4-(2-(N-piperidinyl)ethoxy)phenyl)-5-hydroxy-7-pivaloyloxychrom-3-ene hydrochloride;
3-phenyl-4-(4-(2-(N-piperidinyl)ethoxy)phenyl)-5-methoxy-7-pivaloyloxychrom-3-ene hydrochloride.

The compounds of formula I belong to the class of the Selective Estrogen Receptor Modulators (SERMs) which bind and interact with the estrogen receptor but which act in certain tissues, such as bone, as estrogen agonists and in other tissues, and in particular in the breast and uterus, as estrogen antagonists.

The SERMs retain the beneficial effects of estrogen without some of its side effects

### STATE OF THE ART

In the PCT application WO 94/20098 in the name of Zymogenetics 3,4-diaryl-2H-1-benzopyrans have been disclosed for use in reducing bone loss and in particular bone loss associated with osteoporosis.

A particularly preferred compound for use within the Zymogenetics invention is the 1-enantiomer of centchroman, later developed by Novo Nordisk as levormeloXifene for the treatment of osteoporosis.

Levormeloxifene is hereinafter used as reference compound.

Substituted benzopyran and thiobenzopyran derivatives having a potential anti-estrogenic activity have been later on disclosed in two PCT applications WO 98/25916 and WO 99/65893 in the name of C&C Research Laboratories.

US 5,985,306 discloses the (+)-enantiomers of cis-3,4-chroman derivatives of formula: 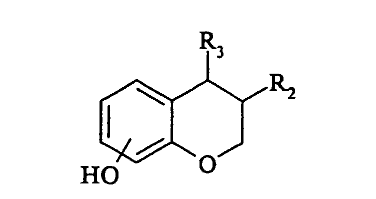 wherein
- R₂: is phenyl optionally substituted with, among others, hydroxy or alkoxy groups and
- R₃: is, among others, phenyl substituted with a -X-(CH₂)ₙ-Y group, wherein X can represent an oxygen atom, n can be 2 and Y can be piperidinyl.

These compounds are useful in the prevention or treatment of estrogen-related diseases or syndromes.

The compounds of the invention differ in their chemical structure from the benzopyrans of the prior art for:
- the presence of a double bond between the atoms in position 3-4
- the presence of an hydroxy group in 5

These differences in the benzopyran structure has revealed of fundamental importance for the pharmacological activity of the compounds of the invention.

J. Med. Chem. 1997, 40, 146-167 reports on a structure-activity relationship study on a series 2-arylbenzothiophene derivatives of Raloxifene and discloses, among others, the following compound 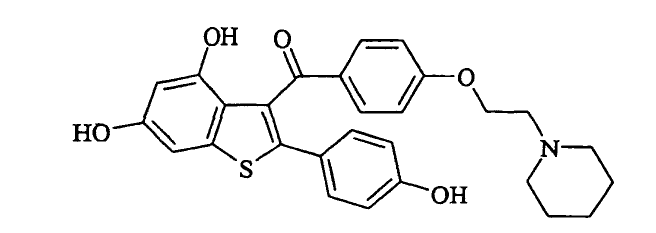 which differs from Raloxifene in that it bears an additional OH group at position 4 of the benzothiophene ring. The authors teach that "the conformation of the ER-ligand complex depends upon the nature of the interacting ligand and that distinct conformation induced by a particular ligand differentially affects which genes are transcriptionally activated", therefore "the agonistic/antagonistic profile for any ER modulator must be determined for each target tissue in question".

A non-steroidal benzothiophene derivative raloxifene, provided with tissue-specific estrogen agonist and antagonist actions has been admitted for clinical use in the US and some European countries.

The compounds of the invention favourably compare also with raloxifene.

Other synthetic compounds with this possible spectrum of activities, including triphenylethylene and dihydronaphtalene have been described as SERMs. However, the development of many of these compounds as drugs has demonstrated to be problematic due to their excessive stimulation of uterine tissue.

The novel SERMs of the invention demonstrate significant beneficial effects on bone and serum lipid levels and antagonistic effects associated with a low degree of intrinsic estrogenicity on reproductive tissue.

For their tissue-specific estrogen-agonistic and antagonistic properties the compounds of the invention can be used in therapy in particular for the prevention and treatment of a number of postmenopausal pathologies, in particular osteoporosis, coronary heart disease and estrogen dependent human cancer.

According to the present invention it is also provided a process for the preparation of a compound of formula (I) 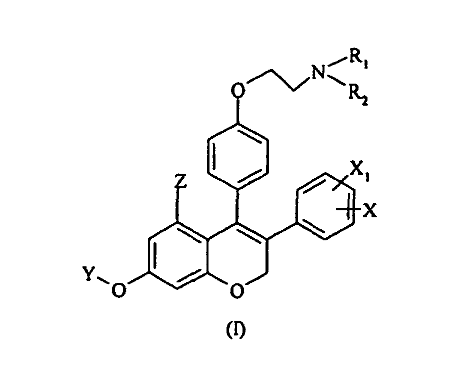 wherein:
- R₁ and R₂,: taken together with the nitrogen atom, form a piperidino or a pyrrolidino ring,
- Y: is hydrogen or a C₁-C₄ alkanoyl or a pivaloyl group,
- Z: is OR₄, wherein R₄ is H or C₁-C₄ alkyl,
- X and X₁: are hydrogen or one is hydrogen and the other is halogen or an OR₃ group wherein R₃ is H, C₁-C₄ alkyl, C₁-C₄ alkanoyl, pivaloyl or benzoyl.

Compounds of formula (I) may be obtained according to scheme (1), by reacting 1,3,5-trihydroxybenzene with a substituted phenylacetonitrile in an aprotic solvent saturated with gaseous HCl, so obtaining the ketimines of formula (II) that can be hydrolyzed in boiling water to give the corresponding ketones of formula (III) that can be cyclized by means of N,N-dimethylformamide dimethylacetal and boron trifluoride diethyl etherate in dimethylformamide (DMF) to give compounds of formula (IV).

Compounds of formula (IV) can be protected in the 7-position by means, for example, of an appropriate anhydride, with or without a solvent, at temperature from 0°C to the boiling point of the solvent or of the pure anhydride, or by means of an appropriate halide, in an aprotic solvent and in presence of a base, so obtaining an ester of formula (V) that have to be stable when submitted to the Grignard reaction.

The so obtained compounds of formula (V) can be hydrogenated, in presence of a suitable catalyst, to give compounds of formula (VI).

The addition of Grignard reagents of formula (VII), obtained from 4-(t-aminoalkyloxy)-1-bromobenzene and Mg in an aprotic solvent, to the ketones of formula (VI), at temperature from -70°C to the boiling point of the solvent, leads to the alcohol (VIII) that can be dehydrated, for example, in an ethanolic solution of conc. HCl, so obtaining compounds of formula (IX).

Compounds of formula (IX) can be alkylated or acylated by means of an alkyl or acyl halide in an appropriate solvent and in presence of a base, or can be acylated by means of an acyl anhydride at high temperature and without a solvent, to give compounds of formula (X) where Y (different from R₄) is the protecting group chosen among those that are stable to the Grignard reaction. Compounds of formula (IX) can be deprotected, for example with conc. HCI or conc. HBr at high temperatures, or, when Y=acyl, with K₂CO₃ in aqueous methanol at room temperature, to give the corresponding free phenols of formula (XI) that can be alkylated, for example by means of an alkyl halide in an appropriate solvent and in presence of a base, or can be acylated, for example by means of an acyl halide in an appropriate solvent and in presence of a base or by means of an acyl anhydride, with or without a solvent, to give compounds of formula (X) where Y is R₄ but different from H.

The preferred protecting group is pivaloyl (2,2-dimethylpropanoyl). 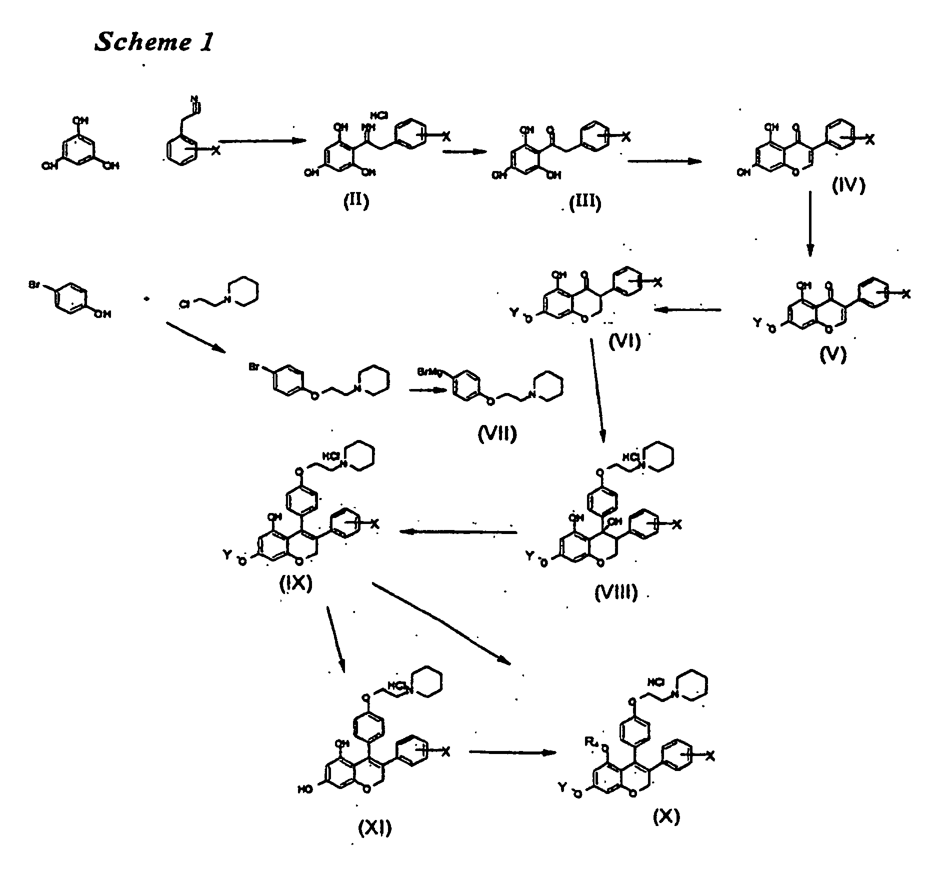 The following examples further illustrate the invention.

### Example 1

### Preparation of 2,4,6-trihydroxyphenyl benzyl ketimine hydrochloride

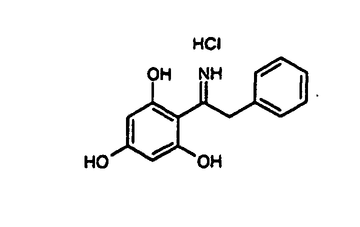

A solution of phloroglucinol (60 g) in diethyl ether (400 ml), refrigerated at 5°C, was added to a solution of phenylacetonitrile (50.1 g) and boron trifluoride diethyl etherate (4.7 ml) in HCl/ETOAc ≅ 5M (800 ml), prepared by bubbling HCl gas in cooled ethyl acetate.

The solution was stored at 5°C for 3 days. The crystalline yellow precipitate was filtered, washed with diethyl ether (50 ml) and used without further purification.
95 g of product were obtained (Yield =80%)
TLC : chloroform/methanol = 90/10, Rf =0.7)

### Example 2

### Preparation of 2,4,6 trihydroxyphenyl benzylketone

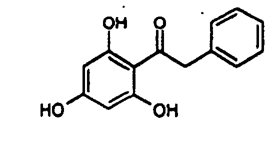

2,4,6-trihydroxyphenyl benzylketimine hydrochloride (95 g) was suspended in water (≅2000 ml) and warmed at 90°C under stirring for 1 hour.

After cooling, a crystalline product was obtained. The solid was filtered, washed with water (200 ml) and dried under vacuum at 60°C.
70 g of product were obtained (yield 85%)
TLC: chloroform/methanol = 90/10, Rf=0.8

### Example 3

### Preparation of 5,7-dihydroxyisoflavone

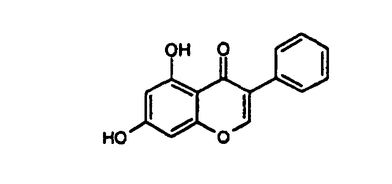

A solution of boron trifluoride diethyl etherate (228 ml) and N,N-dimethylformamide dimethylacetal (84 g) in N,N-dimethylformamide (715 ml) was added to a solution of the compound of example 2 (55 g) in N,N-dimethylformamide (1400 ml), warmed at 50°C. The mixture was heated at 95°C for 60 minutes.

The dark orange solution was cooled at r.t., poured into cold water (10 l) and left overnight without stirring.

The pink solid was filtered, washed with water (1 l) and dried under vacuum at 70°C.
40.3 g of product were obtained (yield 70.4%)
TLC : hexane/ethyl acetate = 70/30, Rf= 0.55

### Example 4

### Preparation of 7-pivaloyloxy-5-hydroxyisoflavone

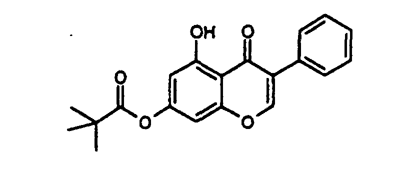

The compound of example 3 (10 g) and pivalic anhydride (50 ml) were heated at 120°C, under magnetic stirring, in a closed flask for 4 h. The red dense solution was kept at r.t. without stirring overnight.

The precipitate was filtered, washed with petroleum ether and dried u.v. at 45°C.
8.9 g of a pink solid were obtained. (yield 65%)
TLC : methylene chloride/methanol=95/5 R.f.=0.8

### Example 5

### Preparation of 3-phenyl-5-hydroxy-7-pivaloyloxychroman-4-one

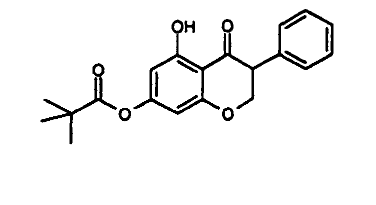

A solution of the compound of example 4 (8.7 g) in acetone (300 ml) was added with 5% Pd/C (9 g) (water content ≅ 50%) and hydrogenated at 40 psi in a Parr apparatus for 2.5 h. The catalyst was filtered on a celite pad and the filtrate evaporated to dryness. The oily residue was dissolved in diethyl ether, dried ( Na₂SO₄) and evaporated again. 8 g of orange oil were obtained (yield 90%).

It was used without further purification.

TLC: petroleum ether/EtOAc = 95/5 R.f.=0.5

### Example 6

### Preparation of 4-(2-(N-piperidinyl)ethoxy)-1-bromobenzene

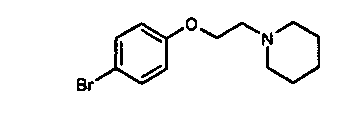

A solution of bromophenol (10 g) in dimethylformamide (160 ml) was added with K₂CO₃ (20.7 g) and heated at 100°C, under stirring, for 10 min. N-(2-chloroethyl)piperidine hydrochloride (9.6 g) was added over a period of 10 min. The mixture was heated and stirred for 2 h, then cooled at r.t., poured into water (300 ml) and extracted with ethyl acetate (300 ml). The organic phase was extracted with 3% HCl (2x200 ml).After alcalinization, the aqueous phase was extracted with ethyl acetate (300 ml); evaporation of the solvent gave 13 g of product as an orange oil.
TLC: methylene chloride/methanol/acetic acid=70/20/10 R.f.=0.7.

### Example 7

### Preparation of 4-(2-(N-piperidinyl)ethoxy)phenylmagnesium bromide

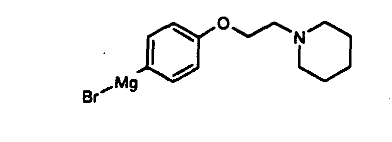

Magnesium (2.1 g) was charged into a 500 ml four necked flask, equipped with a condenser, a dropping funnel and a magnetic stirrer, under N₂ atmosphere.

5 ml of a solution of the compound of example 6 (11.8 g) in freshly distilled tetrahydrofuran (THF) (50 ml) was added, then the mixture was heated to reflux.

The remaining solution was dropped into the boiling reaction mixture over a period of 40 min. The mixture was allowed to reach r.t., then the turbid gray solution was used in the following step.

### Example 8

### Preparation of 3-Phenyl-4-(4-(2-(N-piperidinyl)ethoxy)phenyl)-4,5-dihydroxy-7-pivaloyloxy chromane hydrochloride

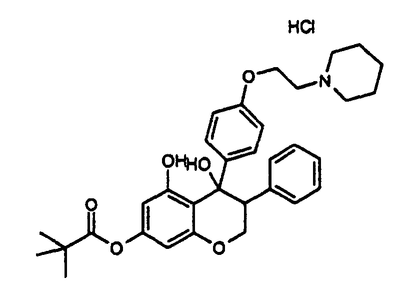

The Grignard solution obtained from the previous step was quickly added to a solution of the compound of example 5 (2.6 g ) in dry THF (20 ml), under stirring. Temperature raised to 40°C. After stirring for 1.5 h, water (10 ml) was added and the mixture evaporated to dryness u.v. The crude product was dissolved in ethyl acetate (300 ml) and the insoluble material filtered off. The organic solution was washed with HCl 0.25N (200 ml) (the hydrochloride salt of the product is more soluble in ethyl acetate than in water), and concentrated to a little volume until precipitation occurred. After ultrasonication, the solid was filtered and air dried.
1.6 g of product were obtained as a white powder. (Yield 36%).
TLC: methylene chloride/methanol = 90/10 R.f.=0.3
M.P.: 220-221°C
NMR: complies.

### Example 9

### Preparation of 3-Phenyl-4-(4-(2-(N-piperidinyl)ethoxy)phenyl)-5-hydroxy-7-pivaloyl oxychrom-3-ene hydrochloride

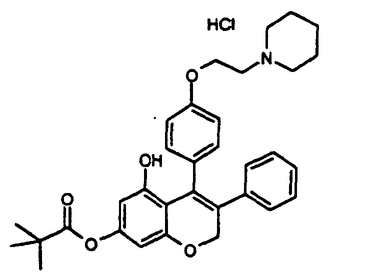

The compound of example 8 (0.71 g) was dissolved in boiling ethyl alcohol (40 ml). The solution was cooled at r.t. and HCl 37% (0.2 ml) was added. After 2 h at r.t, the solution was evaporated to complete dryness and the solid foam was crystallized from acetone. The white solid obtained was filtered, washed with acetone and dried u.v. at 35°C.
550 mg were obtained (yield 80%).
M.P.: 229-230°C
T.L.C.: methylene chloride/methanol/triethylamine = 95/5/1 R.f.=0.3
NMR: complies.

### Example 10

### Preparation of 3-Phenyl-4-(4-(2-(N-piperidinyl)ethoyx)phenyl)-5,7-dihydroxychrom-3-ene hydrochloride

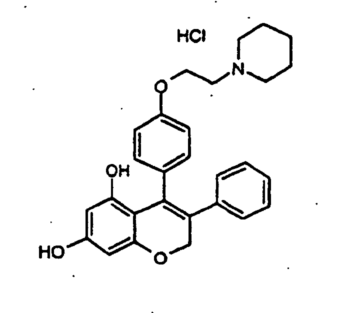

370 mg of the compound of example 9 were dissolved in hot THF (20 ml), then 130 mg of LiAlH₄ were added and the mixture was stirred for 30 min.

Water (170 mg) was cautiously added, the gray solid was filtered off and the solution acidified with HCl/EtOAc. The mixture was evaporated, the residue dissolved in EtOH (2 ml) and ultrasonicated.

A white solid precipitated. It was filtered, washed with Et₂O and dried at 50°C u.v.
210 mg of compound were obtained
M.P. 248-251 °C (Dec.)
T.L.C.: methylene chloride/methanol/triethylamine = 95/5/1. R.f.=0.25
NMR: complies

### Example 11

### Preparation of 4',7-dipivaloyloxy-5-hydroxyisoflavone

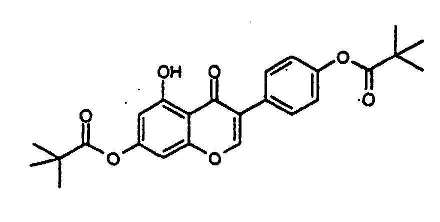

Genisteine (10 g) and pivalic anhydride (75 ml), commercially available or prepared with known methods, were heated at 110°C for 22 h. then the brown solution was cooled and left at r.t. overnight.

A little amount of solid was formed; light petroleum ether (150 ml) was added to the mixture then the abundant solid was filtered, washed with light petroleum ether and dried u.v. at 45°C.
11.6 g of the title compound were obtained.
T.L.C.: light petroleum ether/EtOAc=85/15 R.f. =0.5

### Example 12

### Preparation of 3-(4-pivaloyloxyphenyl)-5-hydroxy-7 pivaloyloxy chroman-4-one

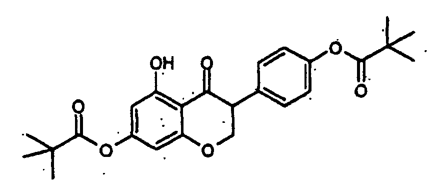

The compound of example 11 (10g) was dissolved in acetone (750 ml) and hydrogenated at 40 psi using 5% Pd/C (10g; 50% H₂O content) as catalyst.

After 3 h the catalyst was filtered off and the solution was evaporated to obtain an oil that was crystallized from light petroleum ether (150 ml)
8.7 g of the title compound were obtained.
T.L.C.: light petroleum ether/EtOAc=85/15. R.f. =0.5

### Example 13

### Preparation of 3-(4-pivaloyloxyphenyl)-4-(4-(2-(N-piperidinyl)ethoxy) phenyl)-4,5-dihydroxy-7-pivaloyloxychromane hydrocloride

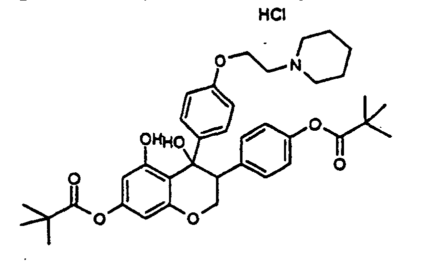

The title compound was obtained with the same procedure described in example 8 in which the compound of example 12 is used in place of the compound of example 5.
T.L.C.: chloroform/methanol /30%NH₄OH=95/5/0.1 R.f.=0.35

### Example 14

### Preparation of 3-(4 pivaloyloxypheayl)-4-(4-(2-(N-piperidinyl)ethoxy) phenyl)-5-hydroxy-7-pivaloyloxychrom-3-ene hydrochloride

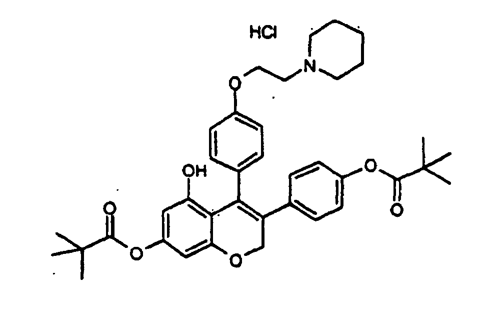

The title compound was obtained with the same procedure described in example 9 in which 3-(4-pivaloyloxyphenyl)-4-(4-(2-(N-piperidinyl)ethoxy) phenyl)-4,5-dihydroxy-7-pivaloyloxy chromane hydrochloride is used in place of the compound of example 8.
T.L.C.: chloroform/methanol /30%NH₄OH= 95/5/0.1 R.f.=0.4
M.P.:243-245°C(Dec.)

### Example 15

### Preparation of 3-(4 pivaloyloxyphenyl)-4-(4-(2-(N-piperidinyl)ethoxy) phenyl)-5-isopropoxy-7-pivaloyloxychrom-3-ene hydrochloride

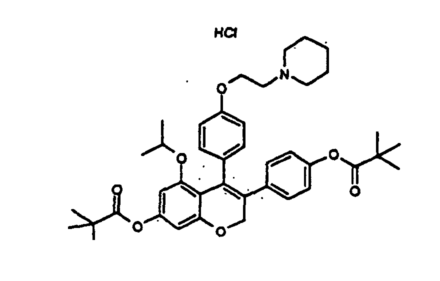

The compound of example 14 (210 mg ) was dissolved in DMF (4 ml), than K₂CO₃ (300 mg) and 2-iodopropane (200 mg) were added and the mixture was stirred at r.t. for 2 h.

. H₂O (5 ml) was added, the precipitated product was filtered, washed with H₂O and purified by flash chromatography over silica gel (eluent: methylene chloride/methanol=95/5 R.f.=0.7).

The product obtained was dissolved in acetone (2 ml) and added with a little excess of HCl in EtOAc so obtaining the crystalline hydrochloric salt.
7 mg of the title compound were obtained.
M.P.: 237-239°C
NMR: complies

### Example 16

### Preparation of 4'-methoxy-7-pivaloyloxy-5-hydroxyisoflavone

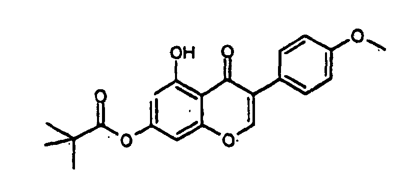

Biochanin A (20 g), commercially available or prepared with known methods, was suspended in pivalic anhydride (100 ml) and the mixture was heated at 120°C, under stirring, for 4 h.

The brown solution was left at r.t. overnight; the precipitated product was filtered, washed with light petroleum ether and dried at 70°C u.v.
19.6 g of the title compound were obtained.
T.L.C.: methylene chloride/methanol =95/5 R.f.=0.8

### Example 17

### Preparation of 3-(4-methoxyphenyl)-5-hydroxy-7-pivaloyloxychroman -4-one

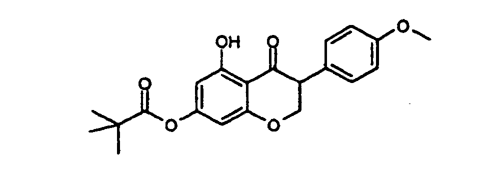

The title compound was obtained with the same procedure described in example 5 in which 4'-methoxy-7-pivaloyloxy-5-hydroxyisoflavone is used in place of the compound of example 4.
TLC: petroleum ether/EtOAc = 95/5 R.f.=0.5

### Example 18

### Preparation of 3-(4-methoxyphenyl)-4-(4-(2-(N-piperidinyl)ethoxy) phenyl)-4,5-dihydroxy-7-pivaloyloxychromane hydrochloride

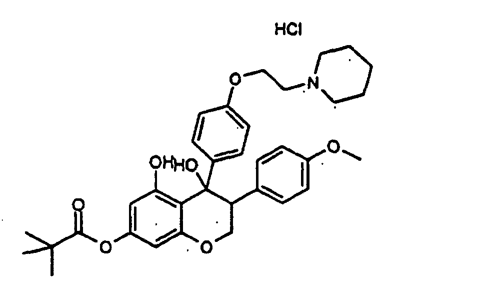

The title compound was obtained with the same procedure described in example 8 in which 3-(4-methoxyphenyl)-5-hydroxy-7-pivaloyloxychroman-4-one is used in place of the compound of example 5.
T.L.C.: chloroform/methanol/30%NH₄OH= 95/5/0.1 R.f.=0.3

### Example 19

### Preparation of 3-(4-methoxyphenyl)-4-(4-(2-(N-piperidinyl)ethoxy) phenyl)-5-hydroxy-7-pivaloyloxychrom-3-ene hydrochloride

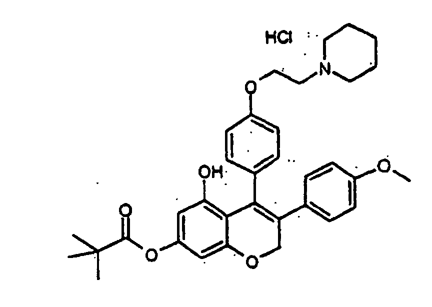

The title compound was obtained with the same procedure described in example 9 in which 3-(4-methoxyphenyl)-4-(4-(2-(N-piperidinyl) ethoxy)phenyl)-4,5-dihydroxy-7-pivaloyloxychromane hydrocloride is used in place of the compound of example 8
T.L.C.: chloroform/methanol/30%NH₄OH= 95/5/0.1 R.f.=0.35
M.P.: 214-216°C (dec.)
NMR: complies

### Example 20

### Preparation of 3-(4-methoxyphenyl)-4-(4-(2-(N-piperidinyl)ethoxy) phenyl)-5,7-dihydroxychrom-3-ene hydrochloride

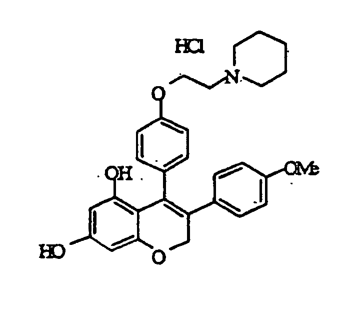

The title compound was obtained with the same procedure described in example 10 in which the compound of examples 19 is used in place of the compound of example 9
T.L.C.: chloroform/methanol/30%NH₄OH= 95/5/1 R.f.=0.3
M.P.:198-200°C(dec.)

### Example 21

### Preparation of 3-phenyl-5-hydroxy-7-methoxychroman-4-one

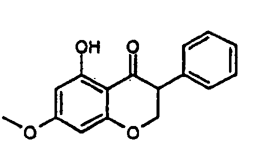

The title compound was obtained with the same procedure described in example 5 in which 5-hydroxy-7-methoxyisoflavone is used in place of the compound of example 4.

### Example 22

### Preparation of 3 phenyl-4-(4-(2-(N-piperidinyl)ethoxy)phenyl)-4,5-dihydroxy-7-methoxychromane hydrochloride

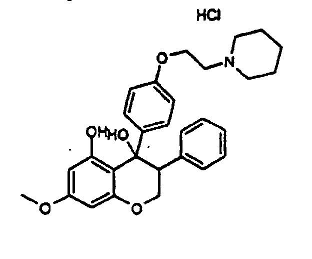

The title compound was obtained with the same procedure described in example 8 in which 3-phenyl-5-hyhoxy-7-methoxychroman-4-one is used in place ofthe compound off example 5.
T.L.C.: methylene chloride/methanol = 90/10 R.f.=0.25

### Example 23

### Preparation of 3-phenyl-4-(4-(2-(N-piperidinyl)ethoxy)phenyl)-5-hydroy-7-methoxychrom-3-ene hydrochloride

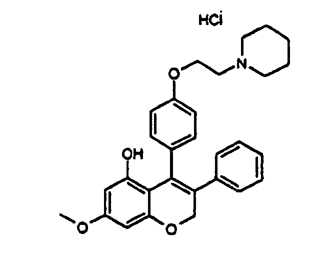

The title compound was obtained with the same procedure described in example 9 in which the compound of example 22 is used in place of the compound of example 8.
T.L.C.: methylene chloride/methanol /30%NH₄OH= 95/5/1 R.f.=0.3
M.P.: 253-256°C (dec.)
NMR: complies

### Example 24

### Preparation of 3-Phenyl-4-(4-(2-(N-piperidinyl)ethoxy)phenyl)-5-methoxy-7-pivaloyloxychrom-3-ene hydrochloride

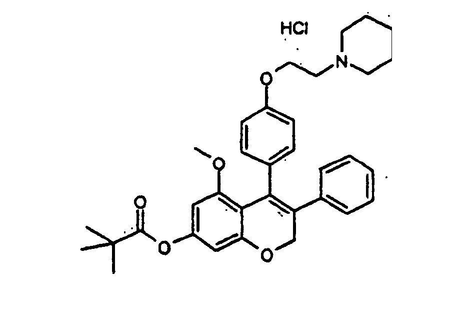

The compound of example 9 (550 mg) was dissolved in DMF (30 ml), then a solution of K₂CO₃ (400 mg) in H₂O (3.5 ml) and a solution of CH₃I (0.9 ml) in DMF (9.5 ml) were added, and the mixture was stirred for 5 h, quenched in H₂O (200 ml) and extracted with EtOAc (200 ml). The organic phase was washed with brine (2x200 ml), dried over sodium sulfate and evaporated u.v. The yellow oil was purified by flash-chromatography over silica gel (eluent: CH₂Cl₂/MeOH/NEt₃=9 5/5/0.1 R.f.=0.4). The product obtained was transformed into its hydrochloride salt by adding HCl/EtOAc to a solution of the free base in EtOAc. 190 mg of white amorphous solid was obtained
T.L.C. : methylene chloride/methanol /NEt3=95/5/0.1 R.f.=0.4
NMR: complies

### Example 25

### Preparation of 3-(4-fluorophenyl)-4-(4-(2-(N-piperidinyl)ethoxy) phenyl)-5-hydroxy-7-pivaloyloxychrom-3-ene hydrochloride

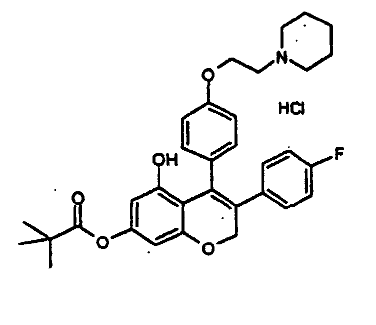

The title compound was obtained following the procedures described in examples 1 to 9 in which the starting material was 4-fluorophenylacetonitrile in place of phenylacetonitrile of example 1.
T.L.C.: methylene chloride/methanol /30%NH₄OH= 95/5/1 R.f.=0.3
M.P.: 198-200°C (dec.)
NMR: complies

### Example 26

### Preparation of 3-phenyl-4-(4-(2-(N-piperidinyl)ethoxy)phenyl)-5,7-diacetyloxychrom-3-ene hydrochloride

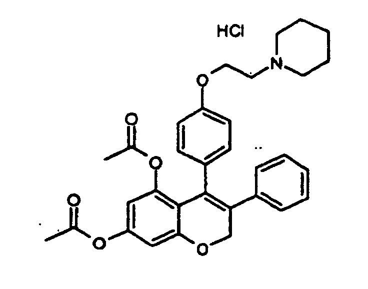

The compound of example 10 (210 mg, prepared as in example 10) was dissolved in acetic anhydride (2 g) at 130°C and stirred for 30 min. The solution was cooled to room temperature, added with ice (2 g), diluted with water (50 ml) treated with aqueous K₂CO₃ and extracted with EfOAc (50 ml). The organic layer was dried over sodium sulfate, filtered, acidified with a slight excess of HCl/EtOAc and evaporated to dryness. The yellow oil so obtained was crystallized from acetone/Et₂O to give 100 mg of the title compound as a light-yellow powder.
T.L.C. methylene chloride/methanol /NEt₃= 95/5/0.1 R.f.=0.55
M.P. 154-156°C (dec.)
NMR: complies

In view of their therapeutic use, the compounds of the invention can be opportunely combined with the usual pharmaceutically acceptable excipients for the preparation of pharmaceutical compositions for parenteral, oral, nasal, rectal, subdermal or transdermal administration according to conventional methods.

One skilled in this art may formulate the compounds in an appropriate manner, and in accordance with accepted practices, such as those disclosed in *Remington's Pharmaceutical Sciences,* Gennaro ed., Mack Publishing Co., Easton, PA, 1990.

## Claims

1. 2H-1-benzopyran derivatives of formula (I) 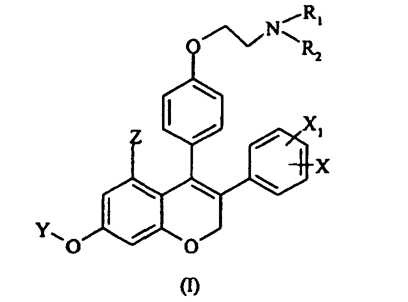 wherein:
R₁ and R₂, taken together with the nitrogen atom, form a piperidino or a pyrrolidino ring,
Y is hydrogen or a C₁-C₄ alkanoyl or a pivaloyl group,
Z is OR₄, wherein R₄ is H or C₁-C₄ alkyl,
X and X₁ are hydrogen or one is hydrogen and the other is halogen or an
OR₃ group wherein R₃ is H, C₁-C₄ alkyl, C₁-C₄ alkanoyl, pivaloyl or benzoyl
and pharmaceutically acceptable salts.

2. 2H-1-benzopyran derivatives according to claim 1 wherein X and X₁ are hydrogen or one is hydrogen and the other is halogen or an OR₃ group in the para position wherein R₃ is as defined in claim 1.

3. One of the following compounds:
3-phenyl-4-(4-(2-(N-piperidinyl)ethoxy)phenyl)-5-hydroxy-7-pivaloyloxychrom-3-ene hydrochloride;
3-(4-methoxyphenyl)-4-(4-(2-(N-piperidinyl)ethoxy)phenyl)-5-hydroxy-7-pivaloyloxychrom-3-ene hydrochloride;
3-phenyl-4-(4-(2-(N-piperidinyl)ethoxy)phenyl)-5-methoxy-7-pivaloyloxychrom-3-ene hydrochloride;

4. Pharmaceutical compositions containing as the active ingredient at least one compound as claimed in claims 1-3 together with pharmaceutically acceptable excipients.

5. The use of the compounds according to claims 1-3 for the preparation of a medicament useful in the prevention and treatment of a number of postmenopausal pathologies, in particular osteoporosis, coronary heart disease and estrogen dependent human cancer.

## Patentansprüche

1. 2H-1-Benzopyranderivate der Formel (I) 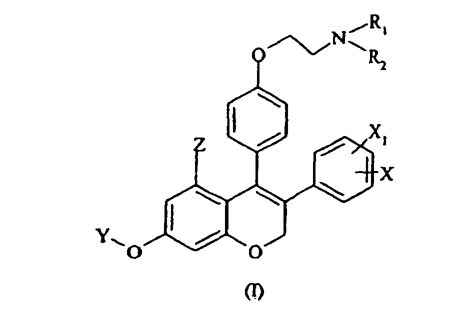 wobei:
R₁ und R₂, zusammen mit dem Stickstoffatom, einen Piperidin- oder einen Pyrrolidinring bilden,
Y Wasserstoff oder eine C₁-C₄-Alkanoyl- oder eine Pivaloylgruppe ist,
Z OR₄ ist, wobei R₄ H oder C₁-C₄-Alkyl ist,
X und X₁ Wasserstoff sind oder eines Wasserstoff ist und das andere Halogen oder eine OR₃-Gruppe, wobei R₃ Wasserstoff, C₁-C₄-Alkyl, C₁-C₄-Alkanoyl, Pivaloyl oder Benzoyl ist, ist
und pharmazeutisch annehmbare Salze.

2. 2H-1-Benzopyranderivate nach Anspruch 1, wobei X und X₁ Wasserstoff sind oder eines Wasserstoff ist und das andere Halogen oder eine OR₃-Gruppe in der para-Position, wobei R₃ wie in Anspruch 1 definiert ist, ist.

3. Eine der folgenden Verbindungen:
3-Phenyl-4-(4-(2-(N-piperidinyl)ethoxy)phenyl)-5-hydroxy-7-pivaloyloxychrom-3-en-hydrochlorid;
3-(4-Methoxyphenyl)-4-(4-(2-(N-piperidinyl)ethoxy)phenyl)-5-hydroxy-7-pivaloyloxychrom-3-en-hydrochlorid;
3-Phenyl-4-(4-(2-(N-piperidinyl)ethoxy)phenyl)-5-methoxy-7-pivaloyloxychrom-3-en-hydrochlorid.

4. Pharmazeutische Zusammensetzungen, die als den aktiven Bestandteil wenigstens eine Verbindung wie in den Ansprüchen 1-3 beansprucht zusammen mit pharmazeutisch annehmbaren Exzipientien enthalten.

5. Verwendung der Verbindungen nach Ansprüchen 1-3 zur Herstellung eines Medikaments, verwendbar bei der Prävention und Behandlung einer Anzahl von nach der Menopause auftretenden Pathologien, insbesondere Osteoporose, koronarer Herzkrankheit und östrogenabhängigem menschlichen Krebs.

## Revendications

1. Dérivés du 2H-1-benzopyrane de formule (I) : 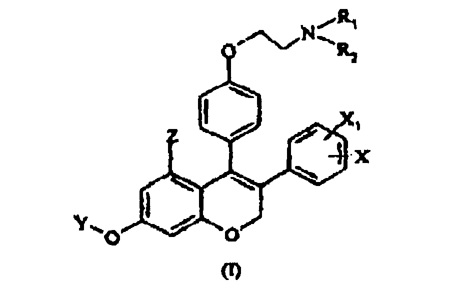 dans laquelle :
R₁ et R₂, pris conjointement avec l'atome d'azote, forment un cycle pipéridino ou un cycle pyrrolidino,
Y est l'hydrogène ou un groupe alcanoyle en C₁-C₄ ou un groupe pivaloyle,
Z est OR₄, où R₄ est H ou un groupe alkyle en C₁-C₄,
X et X₁ sont l'hydrogène ou l'un est l'hydrogène et l'autre est un halogène ou un groupe OR₃, où R₃ est H, un groupe alkyle en C₁-C₄, un groupe alcanoyle en C₁-C₄, pivaloyle ou benzoyle,
et sels acceptables en pharmacie.

2. Dérivés du 2H-1-benzopyrane selon la revendication 1, dans lesquels X et X₁ sont l'hydrogène ou l'un est l'hydrogène et l'autre est un halogène ou un groupe OR₃ en position para, où R₃ est tel que défini dans la revendication 1.

3. L'un des composés suivants :
chlorhydrate de 3-phényl-4-(4-(2-(N-pipéridinyl)éthoxy)phényl)-5-hydroxy-7-pivaloyl-oxychrom-3-ène ;
chlorhydrate de 3-(4-méthoxyphényl)-4-(4-(2-(N-pipéridinyl)éthoxy)phényl)-5-hydroxy-7-pivaloyloxychrom-3-ène ;
chlorhydrate de 3-phényl-4-(4-(2-(N-pipéridinyl)éthoxy)phényl)-5-méthoxy-7-pivaloyl-oxychrom-3-ène ;

4. Compositions pharmaceutiques contenant comme ingrédient actif au moins un composé selon les revendications 1 à 3 conjointement avec des excipients acceptables en pharmacie.

5. Utilisation des composés selon les revendications 1 à 3 pour la préparation d'un médicament utile pour prévenir et traiter un certain nombre de pathologies post-ménopausiques, en particulier l'ostéoporose, la coronaropathie et le cancer oestrogéno-dépendant chez l'humain.
